**Europäisches Patentamt**

**European Patent Office**

**Office européen des brevets**

(11) Veröffentlichungsnummer: **0 245 607 B1**

# EUROPÄISCHE PATENTSCHRIFT

(45) Veröffentlichungstag der Patentschrift: **18.12.91**

(51) Int. Cl.⁵: **A61M 1/00, A61M 16/04, A61M 25/00**

(21) Anmeldenummer: **87103693.5**

(22) Anmeldetag: **13.03.87**

(54) **Doppelkanalsonde zum Einführen in die Luftröhre oder in das Bronchialsystem.**

(30) Priorität: **18.03.86 DE 3608943**
**10.07.86 DE 8600283 U**

(43) Veröffentlichungstag der Anmeldung:
**19.11.87 Patentblatt 87/47**

(45) Bekanntmachung des Hinweises auf die
Patenterteilung:
**18.12.91 Patentblatt 91/51**

(84) Benannte Vertragsstaaten:
**AT BE CH DE ES FR GB GR IT LI LU NL SE**

(56) Entgegenhaltungen:
**EP-A- 0 074 809**
**DE-A- 1 491 652**
**DE-A- 2 364 119**
**FR-A- 2 454 308**
**US-A- 4 300 550**

(73) Patentinhaber: **Schmidt, Christoph**
**Am Kümpel 12**
**W-5300 Bonn 1(DE)**

Patentinhaber: **Schön, Rudolf**
**Im Spichelsfeld 34**
**W-5202 St. Augustin(DE)**

Patentinhaber: **Russ, Jürgen**
**Rosental 32**
**W-5300 Bonn 1(DE)**

(72) Erfinder: **Schmidt, Christoph**
**Am Kümpel 12**
**W-5300 Bonn 1(DE)**
Erfinder: **Schön, Rudolf**
**Im Spichelsfeld 34**
**W-5202 St. Augustin(DE)**
Erfinder: **Russ, Jürgen**
**Rosental 32**
**W-5300 Bonn 1(DE)**

(74) Vertreter: **Müller-Gerbes, Margot**
**Friedrich-Breuer-Strasse 112**
**W-5300 Bonn 3 (Beuel)(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine rohrförmige biegsame Sonde zum Einführen in die Luftröhre, enthaltend ein durchgehendes zum Absaugen ausgebildetes Lumen mit einem am proximalen Eingangsende vorgesehenen Anschlußstück zum Anschließen an eine Absaugvorrichtung und daneben verlaufend ein weiteres Lumen mit gegenüber dem ersten Lumen kleinerem Durchmesser für die Applikation von Medien, wobei die Wandung des kleineren Lumens im Bereich des proximalen Eingangsendes der Sonde eine Ausnehmung aufweist, in die ein elastischer Anschlußschlauch mit einem Ende in das kleinere Lumen eingeführt ist, und an dessen anderem Ende ein Ansatzstück für die Applikation von Medien vorgesehen ist, das distale Ende der Sonde abgerundet ist und ggf. im distalen Endbereich der Sonde in der das große Lumen nach außen begrenzenden Wandung mindestens ein durchgehendes Loch vorgesehen ist. Eine derartige doppellumige Sonde ist als Beatmungstubus zum Einführen in die Luftröhre aus der US-PS 4300 550 bekannt.

Bei der Behandlung von Patienten zur Beatmung und Reanimation sind die folgenden Maßnahmen durchzuführen:

1. Einbringen von Spüllösung in den Tracheal- und Bronchialraum
2. Absaugen von Spüllösung, Schleim, Sekreten, Blut usw. aus dem Tracheal- und Bronchialraum
3. Beatmung und ggf. Sauerstoffzufuhr.
4. Des weiteren ist man neuerdings bemüht, den Patienten auch direkt mit in den Bronchialraum einzubringenden Medikamenten schnell zu versorgen.

Für die vorgenannte Behandlung werden bisher folgende Geräte benutzt:

für Maßnahmen l und 2:

einlumiger Absaugkatheter, wie sie beispielsweise in der DE-AS 23 64 ll9 (US-PS 38 48 604), DE-oS l4 9l 652 (US-PS 33 75 828) oder DE-OS 25 40 536 beschrieben sind.

für Maßnahme 3:

doppellumiger Beatmungstubus oder Ballonkatheter, wie beispielsweise aus der US-PS 43 00 550, DE-OS 28 47 68l und DE-OS 23 08 400 bekannt.

für Maßnahme 4:

gibt es noch kein geeignetes Gerät zum Einbringen von Medikamenten durch die Luftröhre direkt bis tief in die Lunge.

Wenn man hierfür die bekannten einlumigen Absaugkatheter (s. Maßnahmen l und 2) benutzt, so hat das den großen Nachteil, daß die Medikamente erst nach dem Absaugen durch das einzige Lumen eingebracht werden müssen. Infolge des relativ großen Durchmessers des Lumens, das dem Absaugem von Sekret und Schleim dient, gelangen Medikamente nur zu geringen Anteilen bis in die Lunge, im übrigen verbleiben sie im Katheter. Es gelingt nicht, die Medikamente vollständig und tief in die Lunge einzubringen und zu verteilen. Nachteilig ist auch, daß die bekannten Absaugkatheter nicht mit Ansatzstücken zum Ansetzten einer Spritze ausgerüstet sind, was jedoch zum Einbringen von Medikamenten in flüssiger Form erforderlich ist.

Auch die für die Maßnahme 3 verwendeten bekannten Beatmungstuben sind nicht zum Einbringen von Medikamenten durch die Luftröhre bis tief in die Lunge geeignet. Diese bekannten Beatmungstuben weisen einen relativ großen Durchmesser für das Luftvolumen auf und können nur bis in die Trachea eingeführt werden. Bei relativer Kürze der Beatmungstuben würden die eingeführten Medikamente bereits auf dem Wege zur Lunge unterwegs hängenbleiben. Das gewünschte Einführen und Verteilen von Medikamenten bis tief in die Lunge ist nicht möglich.

Der endotracheale Beatmungstubus gemäß DE-OS 23 08 400 weist ein distales abgeschrägtes scharfkantiges Ende auf, das zu erheblichen Verletzungen des Bronchialsystems führen würde und daher nicht bis in die Bronchien eingeführt werden kann.

Bei dem Beatmungstubus gemäß DE-OS 23 08 400 soll neben der Beatmung auch die Absaugung ermöglicht werden, wozu durch den Tubus koaxial ein Schlauch als Katheter geführt ist, der den Tubusquerschnitt verkleinert und behindert. Auch hängt der Schlauch lose in dem großen Lumen des Beatmungstubus und ragt am Ende vor, wodurch keine Kontrolle über seine Lage möglich ist. Mit diesem Beatmungstubus kann nur intermittierend während der Ausatmung abgesaugt werden. Das Spülen und gleichzeitige Absaugen von Sekreten ist mit dem Beatmungstubus gemäß DE-OS 23 08 400 nicht möglich, da bei einer Zuführung der Spülflüssigkeit über den Katheter die Absaugung über den Tubus durchgeführt werden müßte, dabei würde der Beatmungstubus sich an der Bifurkation festsaugen, was diffuse schwerste Blutungen mit größter Lebensgefahr zur Folge hätte. Die notwendige Beatmung bzw. der Sauerstoffaustausch kann dann während des Spülens und Absaugens nicht gleichzeitig durchgeführt werden. Dieser Beatmungstubus ist also auch völlig ungeeignet, um Medikamente tief in die Lunge einzuführen.

Ähnlich verhält es sich mit dem endotrachealen Beatmungstubus gemäß DE-OS 28 47 68l, der auf Grund seines großen Durchmessers für den Luftaustausch (Beatmung), der Kürze des Tubus und des abgeschrägten, scharfkantigen distalen Endes nicht bis tief in das Bronchialsystem eingeführt werden kann und damit nicht für die Applikation von Medikamenten, die in der Lunge verteilt werden sollen, geeignet ist. Soweit die Zuleitungsschläuche für eine zusätzliche Sauerstoffzufuhr sich innerhalb des großen Lumens des Tubus befinden, behindern sie zwar die Beatmung und den Luftaustausch nicht - eine Absaugung von Sekret ist jedoch mit diesem Tubus nicht mehr möglich.

Der aus der US-PS 43 00 550 bekannte endotracheale Beatmungstubus enthält ein durchgehendes großes Lumen zum Absaugen von Luft, dessen proximales Ende an eine Unterdruckeinrichtung angeschlossen ist. Für die gleichzeitige Zufuhr von Sauerstoff enthält der Beatmungstubus ein zweites, kleineres daneben verlaufendes Lumen, dessen distales Ende bzw. distale Austrittsöffnungen weit vor dem distalen Ende des Tubus ausgebildet sind, so daß der austretende Sauerstoff entlang des distalen Tubusbereiches außenseitig geführt ist.

Auch dieser endotracheale Beatmungstubus gemäß US-PS 43 00 550 erweist sich als unbrauchbar für die Applikation von Medikamenten bis tief in die Lunge. Das große Beatmungslumen ist nicht geeignet, weil die Medikamente ohne Strömungsdruck steckenbleiben; bei dem kleinen Lumen hingegen bleiben die Medikamente im Austrittsbereich zwischen Tubus und Luftröhre hängen und gelangen auch wegen der Kürze des Beatmungstubus nicht an den gewünschten Ort.

Aus der Wundbehandlung sind des weiteren doppellumige Drainage-Katheter bekannt geworden, die mit relativ großen Außendurchmessern für ein hohes Absaugvolumen, relativ geringer Länge und abgeschrägter Spitze, wie beispielsweise in der FR-PS 24 54 308 beschrieben, ausgestattet sind. Um eine ausreichende Absaugung zu gewährleisten, weisen diese Drainage-Katheter noch zusätzlich im proximalem Bereich des großen Lumens mit der Außenluft verbindende Löcher auf. Derartige Drainage-Katheter, die bei ihrer Anwendung unter Sicht - d.h. Augenkontrolle - verlegt und gehandhabt werden, sind völlig ungeeignet für die Anwendung im endotrachealen bzw. endobronchialen Bereich. Ihre Bauart würde sofort zu Gewebeschädigungen führen.

Der Erfindung liegt die Aufgabe zugrunde, eine Sonde bzw. einen Katheter zu schaffen, mit dem Medikamente in flüssiger Form durch die Luftröhre bzw. Bronchien tief in das Bronchialsystem bzw. die Lunge eingebracht werden können, und zwar in Verbindung mit einer Absaugung von Sekreten und Schleim oder dergleichen aus dem Bronchialsystem.

Die Erfindung löst die gestellte Aufgabe mit einer rohrförmigen biegsamen doppellumigen Sonde gemäß den kennzeichnenden Merkmalen des Anspruches 1, die so ausgebildet ist, daß sie zum Einführen in das Bronchialsystem und zum alternierenden Einführen Von Medikamenten bzw. Spüllösung mit einer hohen Austrittsgeschwindigkeit am distalen Sondenende durch das kleinere Lumen und Absaugen von Sekret oder dergleichen durch das große Lumen geeignet ist. Die erfindungsgemäße Sonde weist insbesondere die Merkmale auf, daß die Sonde eine Länge von etwa 30 bis 55 cm, einen Außendurchmesser von etwa 5 bis 8 mm hat, das große Lumen für die Absaugung einen Durchmesser von etwa 3 bis 4,5 mm hat und das kleine Lumen einen Durchmesser von etwa 1 bis maximal 2 mm, die beiden Lumen vom proximalen Ende bis zum distalen Ende der Sonde nebeneinander und parallel zur Sondenachse verlaufen, die beiden Lumen nebeneinander am distalen Ende der Sonde münden, wobei das Ansatzstück am Eingangsende des Anschlußschlauches als Ansatzstück für eine Spritze ausgebildet ist.

Der Erfindung liegt die Erkenntnis zugrunde, daß eine möglichst hohe Druckdifferenz zwischen den Enden des Lumens der Sonde notwendig ist, durch die die Medikamente befördert werden sollen, um eine hohe Strömungsgeschwindigkeit zum tiefen Einbringen und Verteilen von Medikamenten in flüssiger Form in die Lunge zu erzielen. Hierfür sind sehr kleine Querschnitte des Lumens im Sinn der Erfindung erforderlich, bevorzugt sind Lumen mit einem Durchmesser von etwa I bis I,5 mm. Um eine feine Verteilung am Ende der Sonde beim Austritt zu erzielen, kann es von Vorteil sein, das kleine Lumen am distalen Ausgangsende leicht düsenförmig zu erweitern.

Des weiteren muß die Sonde geeignet sein, einwandfrei ohne Beschädigung der Schleimhäute und Bronchien in das Bronchialsystem eingeführt zu werden. Hierbei wird auf den Erfahrungen mit einlumigen Kathetern, wie sie zum Absaugen von Schleim und Wasser aus der Lunge bekannt sind, aufgebaut und derartige Sonden erfindungsgemäß mit einem zusätzlichen durchgehenden Lumen mit so kleinem Querschnitt ausgebildet, daß das tiefe Einbringen von Medikamenten in die Lunge mit hoher Strömungsgeschwindigkeit ermöglicht wird. Gleichzeitig wird der Eingang dieses kleinen Lumens mit einem Ansatzstück zum Ansetzen einer Spritze ausgerüstet, wobei das Verbindungsstück als separates Bauteil oder integriert am Ende des Anschlußschlauches ausgebildet sein kann. Das Ansatzstück dient hierbei zum Aufsetzen einer Spritze für das Einführen der flüssigen Medikamente, wobei das Ansatzstück auch beispielsweise noch zusätzlich mit einer Verschlußeinrichtung, wie Ventil, Stöpsel, Verschlußkappe ausgerüstet sein kann.

Die erfindungsgemäße Sonde gestattet in schnellem Wechsel problemlos das alternierende Einführen von Medikamenten durch das kleine Lumen und Absaugen durch das große Lumen. Des weiteren kann gleichzeitig durch das kleine Lumen Spüllösung zugeführt und durch das große Lumen abgesaugt werden. Die erfindungsgemäße Sonde ermöglicht damit mit einem einzigen Gerät eine Mehrzahl von Behandlungen eines Patienten, die die schnelle optimale Versorgung eines Patienten für den Notfall zulassen.

Gemäß der Erfindung weist also das große Lumen der Sonde einen zum Absaugen von Schleim noch ausreichend großen Querschnitt auf und das zusätzliche Lumen mit dem sehr viel kleineren Durchmesser ist noch ausreichend, um die Medikamente direkt bis in die Lunge unter hohem Druckaufbau zu transportieren und zugleich gut zu verteilen. Beide Lumen zusammen dürfen mit ihren Außendurchmessern und Wanddicken den maximal zulässigen Außendurchmesser der Sonde nicht übersteigen, da sonst das Einführen in die Luftröhre behindert ist. Die zulässigen Unterschiede in den Außendurchmessern und der Länge der Sonde sind auf die unterschiedliche Konstitution der Patienten zugeschnitten, beispielsweise Kinder und Erwachsene.

Die beiden Lumen verlaufen am distalen Sondenende achsparallel zur Sondenachse, so daß die Medikamente mit hohem Druck in Achsrichtung weiterströmen und tief in die Lunge unter düsenartiger feiner Verteilung eindringen. Hierbei können die Lumen bündig mit dem distalen Ende der Sonde enden. Es ist auch möglich und ggf. von Vorteil, das kleinere Lumen noch innerhalb der Sonde, wenige Millimeter vor dem distalen Sondenende parallel verlaufend mit dem großen Lumen zu vereinigen und sie gemeinsam am distalen Ende bündig austreten zu lassen. Dies kann zum Beispiel dadurch erfolgen, daß die Zwischenwand zwischen großem und kleinem Lumen etwa 5 bis l5, bevorzugt 5 bis l2 mm vor dem distalen Ende der Sonde endet. Diese Ausbildung der Sonde wird bei Anordnung von Löchern im Bereich des distalen Endes in der Wandung der Sonde, die das große Lumen mit der Außenluft verbinden, bevorzugt, wobei die Löcher in dem vereinigten Bereich der Lumen liegen.

Die Sonde ist aus einem flexiblen biegsamen und sterilisierbaren Material, beispielsweise Kunststoff, hergestellt und weist eine zum Einführen in die Bronchien ausreichende Länge von etwa 30 bis 55 cm auf. Der Durchmesser des großen Lumens zum Absaugen von Schleim beträgt etwa 3 bis 4,5 mm.

Zum sicheren und einwandfreien Einführen der Sonde in die Luftröhre wird weiterhin vorgeschlagen, daß das in das Bronchialsystem einführbare distale Ende abgerundet, bevorzugt mit einer Ringwulst ausgebildet ist, die außenseitig geringfügig über die Wandung der Sonde vorsteht. Damit ist eine gute Führung der Sonde in der Luftröhre erzielbar. Der einseitige Überstand der Ringwulst sollte nicht mehr als etwa 0,4 bis l,4 mm betragen, damit der zulässige Außendurchmesser der Sonde nicht überschritten wird. Dabei kann bei kleinerem Außendurchmesser der größere Überstand gewählt werden.

Die Sonde kann auch mit seitlichen Löchern in der Wandung des großen Lumens in der Nähe des Ringwulstes, d.h. im distalen Endbereich der Sonde, ausgebildet sein, die ein Ansaugen der Sonde an die Schleimhäute verhindern. Diese Löcher brauchen nur einen sehr kleinen Durchmesser von etwa l mm aufzuweisen.

Das proximale Ende der Sonde kann im Eingang des großen Lumens mit einem Anschlußstück für eine Absaugeinrichtung verbunden sein, das beispielsweise eingesteckt wird. Hierfür ist in einer vorteilhaften Weiterbildung der Sonde vorgesehen, daß am proximalen Ende die Wandung der Sonde und die beiden Lumeneingänge, zumindest aber der große Lumeneingang, konisch erweitert sind. Die Anschlußstücke für die Absaugung können ebenfalls mit einem Ventil oder einer Verschließeinrichtung, wie Stöpsel, Verschlußklappe ausgebildet sein. Des weiteren kann die Rohrwandung der Sonde erfindungsgemäß in axialer Längserstreckung mit einem Röntgenkontraststreifen ausgerüstet sein. Das ermöglicht eine Röntgenkontrolle während der Behandlung eines Patienten und Lagekontrolle der Sonde.

Mit der Erfindung wird auf dem in Rede stehenden medizinischen Gebiet ein erheblicher Fortschritt bei der Behandlung von Patienten erreicht. Bisher war es nur möglich, bei der Reanimation und Notfällen mit den vorhandenen Geräten in der folgenden Reihenfolge zu arbeiten:

l) Einführen eines Beatmungstubus in die Trachea

2) Einbringen von Spüllösung in den Beatmungstubus

3) Ventilation mit einem Beatmungsbeutel zur Verteilung der Spülflüssigkeit

4) Einführen eines Saugkatheters mit ausschließlicher Saugung

5) Entfernen des Saugkatheters

6) Einführung eines Cava-Katheters, wobei die Gefahren der Schleimhautverletzung gegeben sind (behelfsmäßige Lösung)

7) Einbringen von Notfallmedikamenten über den Cava-Katheter

8) Entfernen des Cava-Katheters

9) Einsetzen der volumenkontrollierten Beatmung.

Der Zeitaufwand hierfür beträgt insgesamt etwa 3 bis 3 l/2 Minuten.

Bei dieser Therapie ist eine Schädigung durch mangelnde Sauerstoffversorgung des Patienten nicht auszuschließen. Auch ist eine ausreichende Applizierung der Notfallmedikamente durch den Cava-Katheter nicht immer gewährleistet. Hinzu kommt der hohe Materialverbrauch, der arbeitsaufwendig und unwirtschaftlich ist.

Mit der Erfindung wird es möglich, den Patienten wie folgt zu versorgen:

1) Einführen eines Beatmungstubus in die Trachea

2) Einführen der erfindungsgemäßen doppellumigen als Spül- und Saugkatheter ausgebildeten Sonde über die Luftröhre

3) Gleichzeitige Spülung und Absaugung des Bronchialtrakes mit der Sonde, wobei Spüllösung durch das kleine Lumen das Absaugen durch das große Lumen erfolgt,

4) Anschließende tiefe Applikation von Notfallmedikamenten mit der Sonde bis in die Lunge durch das kleine Lumen

5) Entfernen der Sonde

6) Beginn der volumenkontrollierten Beatmung durch den Beatmungstubus.

Zeitdauer der gesamten Behandlung 30 Sekunden bis 1 Minute.

Durch Anwendung der Erfindung wird eine wesentlich schnellere und verkürzte Behandlung des Patienten ermöglicht, die seine Überlebenschancen erheblich steigern. Eine Schädigung des zentralen Nervensystems kann auf ein äußerstes Minimum reduziert werden. Darüber hinaus ist der zu betreibende Arbeitsaufwand wesentlich geringer und auch der Materialeinsatz.

Die Erfindung wird in der Zeichnung an Beispielen erläutert, ist jedoch nicht auf diese beschränkt.

Es zeigen in schematischer Darstellung

Figur 1     Ansicht einer doppellumigen Sonde in Längserstreckung

Figur 2     perspektivische Teilaufsicht auf das distale Ende der Sonde gemäß Figur 1

Figur 3     Ansicht des proximalen Endes einer doppellumigen Sonde

Figur 5 - 7   auszugsweiser Längsschnitt verschieden ausgebildeter distaler Enden einer Sonde

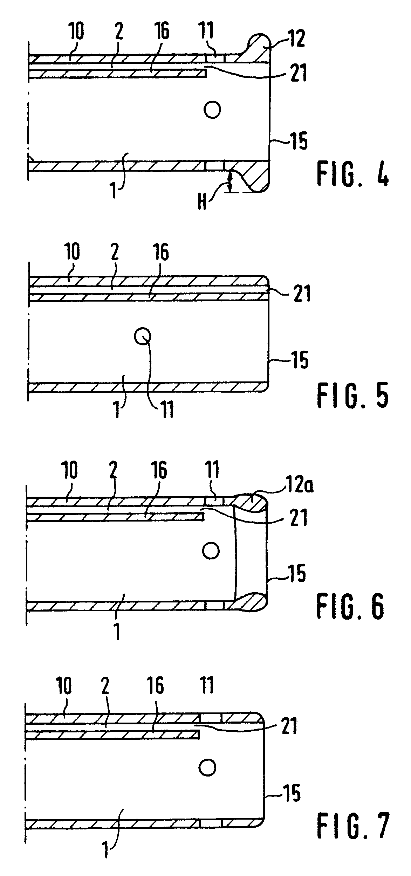

Figur 4     auszugsweiser Längsschnitt des distalen Endes einer doppellumigen Sonde mit Ringwulst.

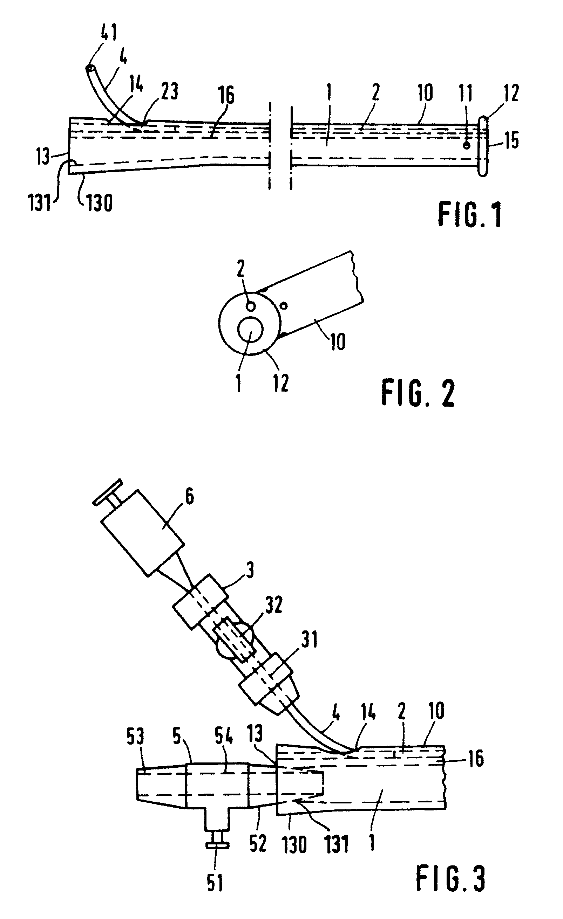

In den Figuren 1 bis 3 ist eine Ausführung der doppellumigen Sonde 10 zum Einführen von Medikamenten in die Lunge und zum Absaugen dargestellt. Die rohrförmige Sonde 10 ist z.B. durch Extrusion aus einem thermoplastischen Kunststoff hergestellt und ist elastisch und biegsam. Die Sonde hat z.B. einen Außendurchmesser von etwa 7 mm. Sie ist mit zwei nebeneinander parallel verlaufenden durchgehenden Lumen 1,2 ausgebildet. Am in die Luftröhre einführbaren distalen Ende 15 der Sonde 10 ist außenseitig der Ringwulst 12 ange-

formt, der dem erleichterten Einführen der Sonde in die Luftröhre dient. Die Sonde weist, bevorzugt im Bereich des distalen Endes vor der Ringwulst 12, Löcher 11 in die das große Lumen 1 begrenzenden Wandung auf, die das Ansaugen der Sonde 10 an die Schleimhäute verhindern sollen. Aus der perspektivischen Ansicht auf das distale Ende der Sonde 10 gemäß Figur 2 ist die Ausbildung und bündige nebeneinanderliegende Mündung der beiden Lumen 1,2 mit unterschiedlichen Durchmessern am distalen Ende zu ersehen. Das große vom proximalen Ende 13 der Sonde bis zum distalen Ende 15 durchgehende Lumen 1 hat einen Durchmesser von etwa 3 bis 5 mm. Durch dieses große Lumen 1 werden Sekrete, Wasser und Schleim oder dergleichen aus der Lunge und den Bronchien abgesaugt. Parallel zu dem großen Lumen 1 verläuft das kleine Lumen 2, die durch die Zwischenwand 16 voneinander getrennt sind. Aus Herstellungsgründen verläuft das kleine Lumen ebenfalls bevorzugt durchgehend vom proximalen bis zum distalen Ende der Sonde. Das kleine Lumen 2 weist einen kleineren Durchmesser von etwa 1 bis maximal 2 mm auf. Das kleine Lumen dient dem Einführen von Medikamenten oder Spüllösungen in die Lunge. Die Sonde nach Fig. 1 ist ein Doppellumen-Katheter für die intrabronchiale Absaugung von Sekret und Applikation von Medikamenten und/oder Spüllösungen. Hierzu wird die Sonde 10, die eine Länge von z.B. 50 cm hat, mit entsprechenden Anschlüssen für das kleine und das große Lumen 2,1 versehen. Die Ausrüstung der Sonde 10 mit Anschlüssen im proximalen Bereich ist beispielhaft in Figur 3 gezeigt. Der proximale Endbereich der Sonde 10 kann beispielsweise mit einer leichten konischen Erweiterung 130 ausgebildet sein, die sowhl die Lumeneingänge als auch die diese begrenzenden Wandungen erfaßt. Auf diese Weise bildet die Eingangsöffnung 131 des großen Lumens einen konischen Trichter zum Einstecken eines Anschlusses für eine Absaugeinrichtung.

Eine Möglichkeit der Zuführung von Medikamenten- oder Spüllösung in das kleine Lumen 2 besteht in der Einführung am proximalen Ende 13 der Sonde direkt über ein Verbindungsstück in das kleine Lumen 2. Eine weitere Möglichkeit, die erfindungsgemäß wegen der größeren Handhabungsfreiheit bevorzugt wird, besteht darin, daß im Bereich des proximalen Endes 13 der Sonde 10, d.h. mit etwas Abstand vom proximalen Ende, die Ausnehmung 14 in der das kleine Lumen begrenzenden Wandung der Sonde ausgebildet ist. Durch diese Ausnehmung 14 kann ein Anschluß, beispielsweise ein elastisches Schlauchstück 4, in den Eingang 23 des kleinen Lumens eingeführt und mit diesem fest verbunden werden. An das elastische Schlauchstück 4 kann am Ende 41 das

Ansatzstück3 für die Spritze 6 angeschlossen werden, oder direkt integriert am Schlauchende 41 angeformt sein, so daß über das Ansatzstück un den Schlauch 4 die Medikamente in das Lumen 2 und damit durch die Luftröhre in die Lunge gebracht werden können.

In der Figur 3 ist schematisch der Anschluß und die Ausbildung von Anschlußstücken 5 für die Absaugung und von Ansatzstücken 3 für die Spritzen 6 eine Sonde gemäß Figur I dargestellt. In dem konisch erweiterten Eingang I3I des Lumens I der Sonde I0 ist das Anschlußstück 5 mit dem konischen Ende 52 eingesteckt. Das Ansatzstück 5 weist die durchgehende Bohrung 54 auf, die mittels des Ventils 5I verschließbar ist. Zum Ansetzen einer Absaugeinrichtung weist das Anschlußstück 5 des weiteren den konischen Zapfen 53 auf.

Für die Einführung von Medikamenten oder Spüllösung mittels einer Spritze 6 in das Lumen 2 ist das freie Schlauchende 4 mit dem Ansatzstück 3 ausgerüstet. Das Ansatzstück 3 weist die durchgehende Bohrung 3I auf, die mittels eines Ventils 32, beispielsweise als Dreiweghahn ausgebildet, verschließbar ist. Das eine Ende des Ansatzstückes 3 ist mit dem Schlauchende 4 verbunden, während an dem anderen Ende die Spritze 6 in die Bohrung 3I einführbar ist. Auch andere Ansatzstücke können verwendet werden.

Mit den erfindungsgemäß ausgebildeten Sonden können also bei der Notversorgung von Menschen Medikamente tief und sicher in das Bronchialsystem und in die Lunge appliziert werden und im gleichen Arbeitsgang bei der Ausführung mit nebeneinander parallel verlaufenden Kanälen zuvor Schleim und Sekrete abgesaugt werden.

Bei Anwendung der erfindungsgemäßen doppellumigen Sonde kann die Spülung und Absaugung auch unter Beatmung, d.h. ohne Abtrennen einer Beatmungseinheit über den Zugang eines rechtwinkligen doppelten Drehkonnektors am Tubus durchgeführt werden.

In der Figur 4 ist in einem auszugweisen Querschnitt die Ausbildung des distalen Endes I5 einer doppellumigen Sonde dargestellt, die am Ende den Ringwulst I2 und die Löcher II aufweist und bei der das kleinere Lumen 2 innerhalb der Sonde I0, bevorzugt noch vor den Löchern II, parallel in das große Lumen mündet. Die Zwischenwand I6 zwischen den Lumen I,2 endet dabei innerhalb der Sonde I0, aber nur in geringem wenige Millimeter umfassenden Abstand vom distalen Ende I5. Die Löcher II können dabei in dem von der Zwischenwand freien Bereich, wo die Lumen vereinigt sind, angeordnet sein.

In den Figuren 5 bis 7 sind weitere vorteilhafte mögliche Ausgestaltungen des distalen Endbereiches der doppellumigen Sonde I0 dargestellt. Bei der Sonde gemäß Figur 5 verlaufen das große und das kleine Lumen I, 2 parallel nebeneinander abgeteilt durch die Zwischenwand I6 bis zum distalen Ende I5 der Sonde I0, die Mündung 2I des kleinen Lumens 2 schließt bündig mit der Mündung des großen Lumens und dem distalen Ende I5 ab. Um ein Ansaugen der Sonde und Anliegen an die Schleimhäute zu verhindern, sind in einem ausreichenden Abstand, der relativ frei wählbar ist, von dem distalen Ende I5 der Sonde mindestens ein Loch II in der Wandung der Sonde vorgesehen, die das große Lumen umgibt. Die Wandungen der Sonde I0 sind am distalen Ende I5 bevorzugt leicht abgerundet ausgebildet. Es ist auch möglich, das kleine Lumen 2 im Mündungsbereich 2I leicht düsenartig zu erweitern, um die Verteilung der eingeführten Medikamente zu fördern.

Bei der Ausführung des distalen Endbereiches der Sonde I0 gemäß Figur 6 ist die Abrundung des Sondenendes, d.h. der Außenwandung, durch eine geringfügige nach innen reichende Verdickung und damit Stabilisierung des Sondenendes erzielt, s. Verdickung I2a. Für diesen Fall ist es ebenfalls zweckmäßig, die Zwischenwand I6 zwischen dem kleinen und dem großen Lumen 2,I einige Millimeter, bis zu etwa I cm vor dem distalen Ende I5 enden zu lassen, so daß das kleine Lumen 2 und das große Lumen I parallel nebeneinander noch im Sondenende münden und gemeinsam aus dem distalen Ende I5 vereinigt austreten. Die Löcher II zur Verhinderung des Ansaugens an den Schleimhäuten können hierbei in dem von der Zwischenwand I6 freien Bereich des distalen Endbereiches der Sonde ringsum gleichmäßig verteilt, beispielsweise zu zweit, dritt oder viert, angeordnet sein. In der Figur 7 ist eine Ausführung eines distalen Endbereichs der Sonde I0, wie in der Figur 6 gezeigt, jedoch ohne verdicktes Ende der Wandung am distalen Ende I5 der Sonde.

## Patentansprüche

1. Rohrförmige biegsame Sonde (10) zum Einführen in die Luftröhre, enthaltend ein durchgehendes zum Absaugen ausgebildetes Lumen (1) mit einem am proximalen Eingangsende (13) vorgesehenen Anschlußstück (5) zum Anschließen an eine Absaugvorrichtung und daneben verlaufend ein weiteres Lumen (2) mit gegenüber dem ersten Lumen kleinerem Durchmesser für die Applikation von Medien, wobei die Wandung des kleineren Lumens (2) im Bereich des proximalen Eingangsendes (13) der Sonde eine Ausnehmung (14) aufweist, in die ein elastischer Anschlußschlauch (4) mit einem Ende in das kleinere Lumen (2) eingeführt ist, und an dessen anderem Ende ein Ansatzstück (3) für die Applikation von Medien vorgesehen ist, das distale Ende der Sonde abgerundet ist und gegebenenfalls im distalen Endbereich der Sonde (10) in der das große Lumen (1) nach außen begrenzenden Wandung mindestens ein durchgehendes Loch (11) vorgesehen ist, **gekennzeichnet durch** die Ausbildung als Spül- und Saugkatheter zum intrabronchialen Absaugen von Sekret oder dergleichen durch das große Lumen (1) und zum alternierenden Einbringen und Verteilen von Medikamenten in flüssiger Form bzw. Spüllösung mit hoher Strömungsgeschwindigkeit bis tief in die Lunge durch das kleinere Lumen (2) mit folgenden Merkmalen:

a) die Sonde (10) hat eine Länge von etwa 30 bis 55 cm
b) die Sonde (10) hat einen Außendurchmesser von etwa 5 bis 8 mm
c) das große Lumen (1 ) für die Absaugung von Sekret hat einen Durchmesser von etwa 3 bis 4,5 mm
d) das kleinere Lumen (2) hat einen Durchmesser von etwa 1 bis maximal 2 mm
e) die beiden Lumen (1,2) verlaufen vom proximalen Ende (13) bis zum distalen Ende (15) der Sonde nebeneinander und parallel zur Sondenachse
f) die beiden Lumen (1,2) münden nebeneinander am distalen Ende (15) der Sonde
g) das Ansatzstück (3) am Eingangsende des Anschlußschlauches ist als Ansatzstück für eine Spritze (6) ausgebildet.

2. Sonde nach Anspruch I,
**dadurch gekennzeichnet,** daß der Durchmesser des kleineren Lumens (2) etwa I bis I I/2 mm beträgt.

3. Sonde nach Anspruch I oder 2,
**dadurch gekennzeichnet,** daß das distale Ende der Sonde mit einer über die die Lumen umschließende Wandung geringfügig überstehenden Ringwulst (I2) ausgebildet ist.

4. Sonde nach Anspruch 3,
**dadurch gekennzeichnet,** daß der Überstand (H) der Ringwulst etwa 0,4 bis I,4 mm beträgt.

5. Sonde nach einem der Ansprüche I bis 4,
**dadurch gekennzeichnet,** daß das Ansatzstück (3) für die Spritze (6) mit dem Anschlußschlauch (4) integriert ausgebildet ist.

6. Sonde nach einem der Ansprüche I bis 5,
**dadurch gekennzeichnet,** daß das Ansatzstück (3) für die Spritze (6) und/oder das Ansatzstück (5) für das Absauglumen (I) mit einer Verschließeinrichtung, wie Ventil, Stöpsel, Verschlußklappe ausgerüstet sind.

7. Sonde nach einem der Ansprüche I bis 6,
**dadurch gekennzeichnet,** daß in axialer Längserstreckung die Wandung der Sonde mit einem Röntgenkontraststreifen ausgerüstet ist.

8. Sonde nach einem der Ansprüche I bis 7,
**dadurch gekennzeichnet,** daß die die beiden Lumen voneinander trennende Zwischenwand (I6) innerhalb der Sonde kurz vor dem distalen Ende (I5) der Sonde endet.

9. Sonde nach Anspruch 8,
**dadurch gekennzeichnet,** daß die Zwischenwand etwa 5 mm bis I2 mm vor dem distalen Ende (I5) endet.

10. Sonde nach einem der Ansprüche I bis 9,
**dadurch gekennzeichnet,** daß das distale Ausgangsende (2I) des kleineren Lumens (2) düsenförmig erweitert ist.

11. Sonde nach einem der Ansprüche I bis I0,
**dadurch gekennzeichnet,** daß am proximalen Ende (I3) der Sonde die Wandung der Sonde und die Lumeneingänge konisch erweitert sind.

**Claims**

1. Tubular flexible probe (10) for insertion into the windpipe, containing a lumen (1) passing therethrough, which is formed for suction purposes, with a connection piece (5) provided at the proximal inlet end (13) for connection to a suction arrangement, and running next to it a further lumen (2) with a smaller diameter compared with the first lumen, for the administration of media, wherein the wall of the smaller lumen (2), in the region of the proximal inlet end (13) of the probe, has a recess (14) into which an elastic connection hose (4) is inserted with one end in the smaller lumen (2) and at the other end of said hose there is provided an attachment piece (3) for the administration of media, the distal end of the probe is rounded off and, if applicable, in the distal end region of the probe (10) there is provided in the wall externally delimiting the large lumen (1) at least one hole (11) passing therethrough, characterised by being formed as a rinsing and suction catheter for intrabronchial drawing off of secretion or the like through the large lumen (1) and for the alternating introduction and distribution, through the smaller lumen (2), deep into the lung, with high flow speed, of medicaments in the fluid form and of rinsing solution respectively, and having the following features:

    a) the probe (10) has a length of approximately 30 to 55 cm;

    b) the probe (10) has an outside diameter of approximately 5 to 8 mm;

    c) the large lumen (1) for the drawing off of secretion has a diameter of approximately 3 to 4.5 mm;

    d) the smaller lumen (2) has a diameter of approximately 1 to a maximum of 2 mm,

    e) the two lumina (1, 2) run from the proximal end (13) as far as the distal end (15) of the probe side by side and parallel to the probe axis;

    f) the two lumina (1, 2) end side by side at the distal end (15) of the probe;

    g) the attachment piece (3) at the inlet end of the connection hose is formed as an attachment piece for a syringe (6).

2. Probe according to claim 1, characterised in that the diameter of the smaller lumen (2) amounts to approximately 1 to 1 1/2 mm.

3. Probe according to claim 1 or 2, characterised in that the distal end of the probe is formed with an annular bead (12) which projects slightly over the wall enclosing the lumina.

4. Probe according to claim 3, characterised in that the projection (H) of the annular bead amounts to approximately 0.4 to 1.4 mm.

5. Probe according to one of the claims 1 to 4, characterised in that the attachment piece (3) for the syringe (6) is formed so as to be integrated with the connection hose (4).

6. Probe according to one of the claims 1 to 5, characterised in that the attachment piece (3) for the syringe (6) and/or the attachment piece (5) for the suction lumen (1) are fitted with a closing device, such as valve, stopper, closure flap.

7. Probe according to one of the claims 1 to 6, characterised in that the wall of the probe is fitted, in the axial longitudinal extension, with an X-ray contrast strip.

8. Probe according to one of the claims 1 to 7, characterised in that the intermediate wall (16) separating the two lumina from each other ends within the probe shortly before the distal end (15) of the probe.

9. Probe according to claim 8, characterised in that the intermediate wall ends approximately 5 mm to 12 mm before the distal end (15).

10. Probe according to one of the claims 1 to 9, characterised in that the distal outlet end (21) of the smaller lumen (2) is extended in the form of a nozzle.

11. Probe according to one of the claims 1 to 10, characterised in that at the proximal end (13) of the probe, the wall of the probe and the lumen inlets are extended conically.

## Revendications

1. Sonde tubulaire flexible (10) à introduire dans la trachée, contenant un conduit (1) continu formé en vue de l'aspiration, avec une pièce de raccordement (5) prévue à l'extrémité proximale d'entrée (13) en vue du raccordement à un dispositif d'aspiration, et s'étendant à côté un autre conduit (2) d'un diamètre inférieur à celui du premier conduit, en vue de l'administration de médias, la paroi du plus petit conduit (2) présentant au voisinage de l'extrémité proximale d'entrée (13) de la sonde une découpe (14) par laquelle un tube de raccordement (4) élastique est introduit par une extrémité dans le petit conduit (2), un embout (3) étant prévu à son autre extrémité en vue de l'administration de médias, l'extrémité distale de la sonde étant arrondie, tandis que le cas échéant au moins un orifice de traversée (11) est prévu dans le grand conduit (1) au voisinage de l'extrémité distale de la sonde (10) dans la paroi délimitant vers l'extérieur le grand conduit (1), **caractérisée par**

    - une réalisation en forme de cathéter de rinçage et d'aspiration, en vue de l'aspiration intrabronchiale de sécrétion ou similaires à travers le grand conduit (1) et en vue de l'introduction et la distribution alternées de médicaments sous forme liquide ou d'une solution de rinçage, avec une vitesse d'écoulement élevée, jusqu'à profondément dans les poumons, à travers le petit conduit (2), et avec les caractéristiques suivantes:

    a) la sonde (10) possède une longueur d'environ 30 à 55 cm

    b) la sonde (10) possède un diamètre extérieur d'environ 5 à 8 mm

    c) le grand conduit (1) d'aspiration de sécrétion possède un diamètre d'environ 3 à 4,5 mm

    d) le petit conduit (2) possède un diamètre d'environ 1 à au plus 2 mm

e) les deux conduits (1,2) s'étendent depuis l'extrémité proximale d'entrée (13) jusqu'à l'extrémité distale (15) de la sonde, l'un à côté de l'autre et parallèlement à l'axe de la sonde

f) les deux conduits (1.2) débouchent l'un à côté de l'autre à l'extrémité distale (15) de la sonde g) l'embout (3) à l'extrémité d'entrée du tube de raccordement est réalisé sous forme d'un embout pour une seringue (6).

2. Sonde selon la revendication 1, **caractérisée en ce que**

- le diamètre du petit conduit (2) s'élève à environ 1 à 1,5 mm.

3. Sonde selon la revendication 1 ou 2, **caractérisée en ce que**

- l'extrémité distale (15) de la sonde est réalisée avec un bourrelet annulaire (12) débordant un peu de la paroi entourant le conduit.

4. Sonde selon la revendication 3, **caractérisée en ce que**

- le débordement (H) du bourrelet annulaire (12) s'élève de 0,4 à 1,4 mm.

5. Sonde selon l'une des revendications 1 à 4, **caractérisée en ce que**

- l'embout (3) pour la seringue (6) est réalisé sous une forme intégrant le tube de raccordement (4).

6. Sonde selon l'une des revendications 1 à 85, **caractérisée en ce que**

- l'embout (3) pour la seringue (6) et/ou l'embout (5) pour le conduit d'aspiration (1) sont équipés d'un dispositif de fermeture tel qu'une vanne, un bouchon ou une clapet de fermeture.

7. Sonde selon l'une des revendications 1 à 6, **caractérisée en ce que**

- dans la direction longitudinale axiale, la paroi de la sonde est munie d'une bande à contraste Röntgen.

8. Sonde selon l'une des revendications 1 à 7, **caractérisée en ce que**

- la paroi intermédiaire (16) séparant l'un de l'autre les deux conduits à l'intérieur de la sonde se termine peu avant l'extrémité distale (15) de la sonde.

9. Sonde selon la revendication 8, **caractérisée en ce que**

- la paroi intermédiaire (16) se termine environ 5 à 12 mm avant l'extrémité distale (15) de la sonde.

10. Sonde selon l'une des revendications 1 à 9, **caractérisée en ce que**

- l'extrémité distale de sortie (21) du petit conduit (2) est élargie en forme de buse.

11. Sonde selon l'une des revendications 1 à 10, **caractérisée en ce qu'**

- à l'extrémité proximale (13) de la sonde la paroi de la sonde et les entrées des conduits sont coniquement élargies.

FIG.1

FIG. 2

FIG.3

10  2  16  11  12
21
15

**FIG. 4**

H

1

10  2  16
21
15

**FIG. 5**

1  11

10  2  16  11  12a
21
15

**FIG. 6**

1

10  2  16  11
21
15

**FIG. 7**

1